# EUROPEAN PATENT SPECIFICATION

(11) **EP 0 565 525 B1**
(45) Date of publication and mention of the grant of the patent: **29.10.1997**
(21) Application number: 91917406.0
(22) Date of filing: 31.12.1990
(51) Int. Cl.: A01G 1/04, A01H 1/00, C12N 1/14

(54) **INOCULUM FROM ECTOMYCORRHIZAL FUNGI FORMING ENDOMYCORRHIZAL INFECTION WITH HERBACEOUS PLANTS**
INOKULUM VON ECTOMYCORRHIZA-PILZEN DIE EINE ENDOMYCORRHIZA-INFEKTION BEI GRÄSERN BEWIRKEN
INOCULATION DE CHAMPIGNONS DU TYPE ECTOMYCORRHIZAE PRODUISANT UNE INFECTION ENDOMYCORRHIZALE DE PLANTES HERBACEES

(43) Date of publication of application: 20.10.1993
(73) Proprietor: JANERETTE, Carol A., Glenside, PA 19038 (US)
(72) Inventor: JANERETTE, Carol A., Glenside, PA 19038 (US)
(74) Representative: Dowden, Marina
(86) International application number: US9007701
(87) International publication number: WO9211752

(56) References cited:
- US-A- 4 327 181
- Forest Science, Volume 22, No. 3, issued September 1975, MARX et al., "Growth and Ectomycorrhizal Development of Loblolly Pine Seedlings in Fumigated Soil Infested with the Fungal Symbiont Pisolithus tinctorius," pages 245-254, see page 246.
- The New Phytologist, Volume 92, issued 1982, PICHE et al., "Development of Mycorrhizae, Extramaterial Mycelium and Sclerotia on Pinus strobus Seedlings," pages 211-220, see pages 212, 217 and 218.
- The New Phytologist, Volume 87, issued 1981, DANIELS et al., "Evaluation of the Commercial Potential of the Vesicular-Arbuscular Mycorrhizal Fungus, Glomus epigaeus," pages 345-354, see pages 346 and 347.

## Description

### Field of the Invention

This invention relates to mycology, more specifically to a process for the production of inoculants for herbaceous plants and to the inoculants so produced.

### Background of the Invention

The nomenclature used in this application is intended to be consistent with Snell and Dick, A Glossary of Mycology, Harvard U. Press, Cambridge, MA (1957).

Mycorrhizae are symbiotic associations between the hyphae of certain fungi and the absorbing organs of plants, typically the roots. Mycorrhizal fungi are classified according to the manner in which they infect roots. The two main types are ectomycorrhizae in which fungal hyphae penetrate the intercellular spaces between root cells without entering the interior of the cells, and endomycorrhizae where projections of the fungus enter the interior of the cell.

Ectomycorrhizae are generally associated with trees and other woody species and are formed by "higher fungi" that are found in a number of families of basidiomycetes and ascomycetes. Roots infected with ectomycorrhizal fungi are different from uninfected roots. They are short, swollen, branched and lack root hairs.

Endomycorrhizae are generally associated with herbaceous plants such as grasses, corn, onions and many more, however there are some trees that also form endomycorrhizae. Endomycorrhizae are formed from spores produced by "lower fungi," classified as zygomycetes, and belong to one family, the Endogonales. The fungi that produce these spores are unknown. Superficially, the infected roots look normal, however, the infection can be detected by microscopic examination. Such examination shows the hyphal projections of the fungus that have invaded the cells. These hyphae may have small branches (arbuscles) or swellings at their tips (vesicles). Many endomycorrhizae are called "Vesicular-Arbuscular Mycorrhizae" or VAM, because of the presence of vesicles and arbuscles inside root cells. Ectomycorrhizae generally do not have vesicles or arbuscles.

Ectomycorrhizal fungal inoculants for woody plants such as pine have been produced. See, for example, The new phytologist, Volume 92, issued 1982, Piché et al., "Development of Mycorrhizae, Extra matrical Mycelium and Sclerotia on Pinus Strobus seedlings," pages 211-220, and Litchfield et al., U.S. Pat. 4,327,181, "Aerobic Submerged Fermentation of Sporulating Ectomycorrhizal Fungi" (1982) disclosing liquid culture of selected fungi to produce inoculants for broadcast over forest soil. Marx et al., "Growth and Ectomycorrhizal Development of Loblolly Pine Seedlings in Fumigated Soil Infested with the Fungal Symbiot Pisolithus Tinctorium," Forest Science Vol. 22, pp. 245-254 (1975), show the use of Pisolithus Tinctorius cultured in an agar/vermiculite/peat moss medium in forest nurseries. See also Mosse et al., U.S. Pat. 4,294,037, "Production of Mycorrhizal Fungi" (1981) and Warner, U.S. Pat. 4,551,165, "Mycorrhizal Seed Pellets" (1985).

As far as known, however, inoculants suitable for herbaceous plants such as wheat or the common vegetables corn, onion, asparagus and the like have not heretofore been produced. Compare Watrud, "Spore Germination and Axenic Culture of Endomycorrhizae," writing at page 81 of Methods and Principles of Mycorrhizal Research, the American Phytopathological Society (1982): ". . . To date, successful axenic subculture of hyphae of vesicular-arbuscular mycorrhizae has yet to be reported. . . ." See also Hudson, Fungal Biology, pp. 218 and 219, Edward Arnold (1986); Smith and Douglas, The Biology of Symbiosis, pp. 152 and 153, Edward Arnold (1987); and Mugnier et al., U.S. Patent 4,599,312, "Method of Producing Endomycorrhizian Fungi with Arbuscules and Vesicles in Vitro" (1986).

### DETAILED DESCRIPTION OF THE INVENTION

It has now been found that fungal inoculants for herbaceous plants can be made by a versatile process in which mycelia of selected ectomycorrhizal fungi are initially grown from cultures on a solid medium. These mycelia, still in the solid medium (fungal plugs), are then axenically added to perlite wetted with a nutrient solution and incubated in vitro. The incubation is conducted under the conditions described below for a period of time sufficient to allow the formation of sclerotia, microsclerotia or the initials of sclerotia or microsclerotia. Generally, the incubation is conducted for between about 2 and about 4 months, preferably about 3 months. The inoculant obtained can be used with woody plants, with which the inoculant generally produces symbiotic associations having ectomycorrhizal morphology. The inoculant can also be used with herbaceous plants, with which the inoculant generally produces symbiotic associations having endomycorrhizal morphology.

The solid medium used in the initial growth of mycelia is generally gelled agar. Modess Modification of Hagem Agar in the agar can be the nutrient. Other nutrients, such as a modified Melin and Rama Das Agar, can also be used. Three or four weeks in the dark at around 25°C provide sufficient time for initial growth.

Fungal plugs taken from the agar growth medium are axenically added to jars containing perlite and nutrient solution and incubated in vitro for about three months in the dark under quiescent and substantially anaerobic conditions. In this connection, "quiescent" simply means without agitation. A useful nutrient solution is Fowells and Krauss's pine nutrient solution modified by the addition of glucose and thiamine (Fowells and Krauss, "The inorganic nutrition of Loblolly pine and Virginia pine with special reference to nitrogen and phosphorus" Forest Science 5:95-112 (1959)).

It should be noted that the inert carrier used in the nutrient solution is somewhat selective. The most satisfactory results have been obtained with perlite as an effective carrier although some modified clays can be used. Vermiculite has been found ineffective under the conditions disclosed. The proportion of carrier to liquid nutrient (carrier/nutrient weight ratio) is not critical but around at least 2/1 has been found satisfactory to provide an effective saturated support.

The inoculum prepared above can be used with herbaceous plants or by broadcast sowing with woody plants. In the former case, one axenically germinated seed can be placed on top of one teaspoon full of the fungus inoculum about three centimeters below the surface of a flower pot filled with perlite. The plants are then grown in the greenhouse for four months. The pots are saturated twice weekly with a low-phosphorus nutrient solution (Peter's Lo Phos Fertilizer).

Known ectomycorrhizal fungi that formed mycorrhizal infections having endomycorrhizal morphology with corn, wheat, onion, and/or asparagus plants include the following:

**TABLE I**

| | Name | Source |
|---|---|---|
| 1. | Rhizopogon roseolus (Melhuish #20) | John Melhuish U.S.D.A. Forest Service |
| 2 . | Pisolithus tinctorius (ATCC #38054) | American Type Culture Collection |
| 3. | Amanita muscaria (Melhuish #21) | John Melhuish U.S.D.A. Forest Service |
| 4. | Astraeus hygrometricus (ATCC #46449) | American Type Culture Collection |
| 5. | Cenococcum geophilum (ATCC #38052) | American Type Culture Collection |
| 6. | Scleroderma aurantium (ATCC #58507) | American Type Culture Collection |
| 7. | Athelia neuhoffii (Melhuish #47) | John Melhuish U.S.D.A. Forest Service |
| 8. | Boletinellus merulioides (Melhuish #64) | John Melhuish U.S.D.A. Forest Service |
| 9. | Hebeloma anthracophilum (Melhuish #54) | John Melhuish U.S.D.A. Forest Service |
| 10. | Hebeloma crustuliniforme (Melhuish #53) | John Melhuish U.S.D.A. Forest Service |
| 11. | Paxillus involutus (ATCC #46218) | American Type Culture Collection |
| 12. | Piloderma bicolor (Melhuish #50) | John Melhuish U.S.D.A. Forest Service |
| 13. | Rhizopogon nigrescens (Melhuish #38) | John Melhuish U.S.D.A. Forest Service |
| 14. | Scleroderma albidum (ATCC #58021) | American Type Culture Collection |
| 15. | Scleroderma polyrhizum (Melhuish #68) | John Melhuish U.S.D.A. Forest Service |
| 16. | Suillus cothurnatus (Melhuish #31) | John Melhuish U.S.D.A. Forest Service |
| 17. | Alpova pachyploeus (Marx #258) | Dr. Donald Marx U.S.D.A. Forest Service |
| 18. | Boletus punctipes (Melhuish #15) | John Melhuish U.S.D.A. Forest Service |
| 19. | Lactarius deliciosus (ATCC #36647) | American Type Culture Collection |

While the reasons for the efficacy of the present inoculun and its preparation are not completely understood, it can theoretically be explained as follows. Fungi are able to propagate from a number of sources or propagules including spores, infected root fragments, mycelia, hyphae, etc. One class of propagule are sclerotia (Willets, "Sclerotium Formation," Filamentous Fungi 3:197-213 (1978)), and these are believed to be involved here. Many sclerotia are too small to be seen by the naked eye but they can be seen with a microscope. However, some sclerotia are observable with the unaided eye. Microsclerotia are also believed to be involved (Baard et al. "Structure and Lysis of Microsclerotia . . .," Trans. Br. Mycol. Soc. 77(2):251-260 (1981)). The development of the mycelium in contact with the inert support and nutrient thus proceeds until sclerotia, microsclerotia, or their initials are produced. The initials of microsclerotia are recognizable under the microscope as enlarged areas along fungal hyphae. Microsclerotia are believed to develop from these swollen structures and may be considered a dimorphic form of the fungus. Under some environmental conditions, the fungus has long, thin, hair-like hyphae, while under other conditions the fungus may have the more rounded morphology of microsclerotia. Operable fungi for the present inventions are those fungi which produce sclerotia or microsclerotia. Spore-like structures may also be found in the inoculant.

The inoculant of the present invention has good shelf life. When stored at 24°C in the dark, inocula of the present invention have been found to remain effective for in excess of one year, usually for in excess of three years.

The following nonlimiting examples illustrate the invention.

### EXAMPLES 1-6

### A. Preparation of Inoculum

Selected fungi (Cenococcum Geophilum, Pisolithus tinctorius, Astraeus hygrometricus Amanita muscaria, Rhizopogon roseolus, and Scleroderma aurantium) were axenically grown from mycelia for three weeks at 24°C in the dark in petri plates on Hagem's nutrient agar modified by Modess (Modess, 0., 1941, Zur Kenntnis der Mykorrhizabildner von Kiefer und Fichte, Symbolae Bot. Upsalienses 5(1): 1-146) as shown in Table II.

**Table II**

| Formulation* of Modess Modification of Hagem Agar | |
|---|---|
| KH₂PO | 0.5 g |
| MgS0₄.7H₂O | 0.5 g |
| NH₄Cl | 0.5 g |
| FeCl₃ (1% solution) | 1.0 ml |
| Glucose | 5.0 g |
| Malt extract | 5.0 g |
| Agar | 10.0 g |
| H₂O (distilled) | 1000.0 ml |

| | |
|---|---|
| * Final pH = 4.7 | |

Subsequently, 0.5 cm plugs were cut from the periphery of the fungal colonies and axenically added to separate test tubes containing 40 ml (8 g) of strained perlite and 20 ml of Fowells and Krauss's modified nutrient solution (Table II). The test tubes containing the perlite and nutrient solution had previously been autoclaved at 120°C and 15 lbs pressure for 20 minutes. Inoculated test tubes were incubated for three months in the dark at 24°C.

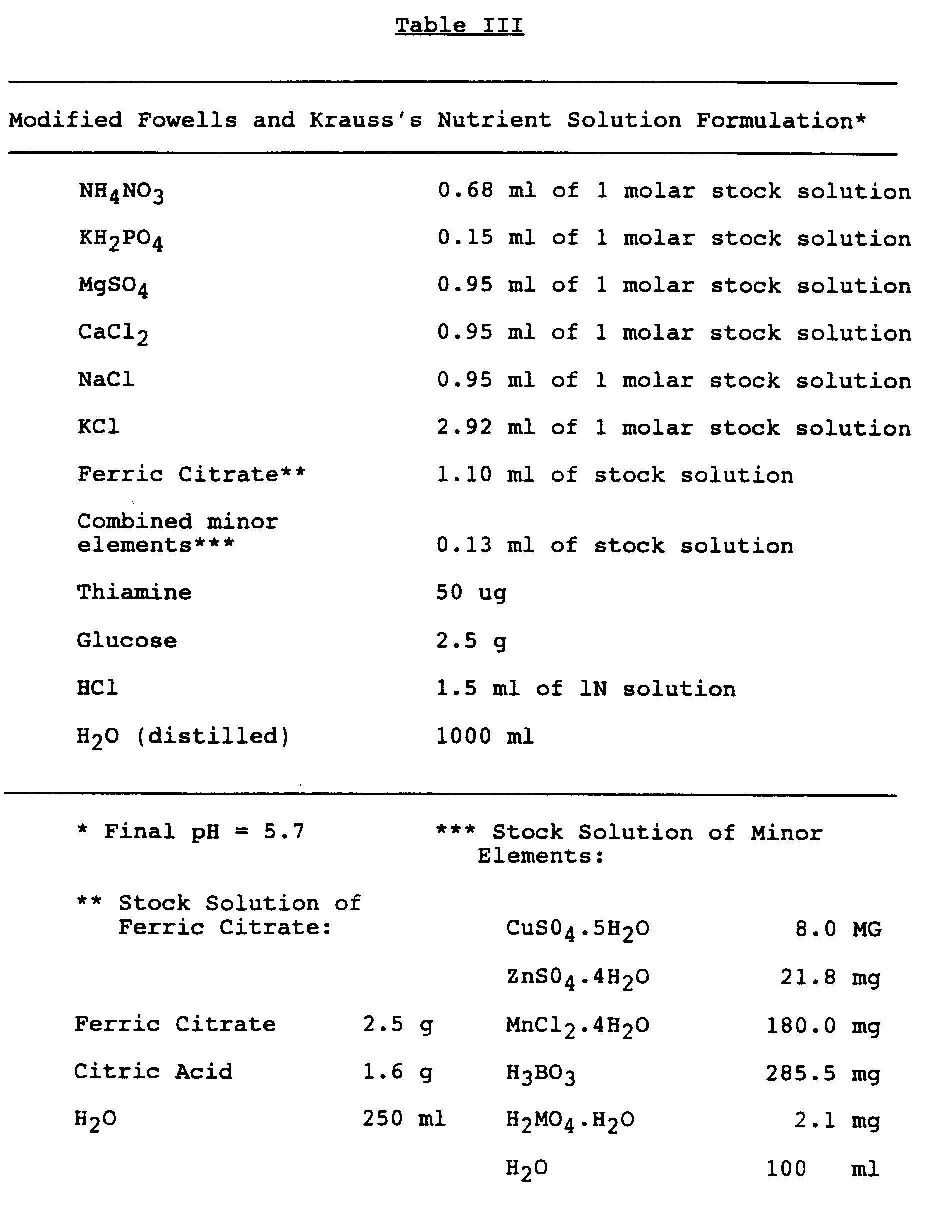

Five ml of Fowells and Krauss's nutrient solution was added to each test Tube and thoroughly mixed with the incubated inoculum before use.

### B. Growth of Endomycorrhizal Structures in Herbaceous Plants

Seeds of onion, wheat, corn and asparagus that had been soaked in distilled water overnight were washed for 1 minute in an aqueous solution containing 1% HgCl₂ and 1 ml of Tween 20 per liter and rinsed three times with sterile distilled water. Seeds were then axenically placed in sterile petri plates containing moistened filter paper and allowed to germinate in the dark at 24°C. When the emerged radicles were between 1 cm and 3 cm long (within 1 week for all seeds), they were planted in the greenhouse in 4-inch pots filled with strained horticultural grade perlite. A hole was dug 3 cm from the surface, and one teaspoon full of the fungus inoculum was put in the hole with one germinated seed placed on top of the inoculum and covered with perlite. Twenty pots were prepared for each fungus being tested along with twenty control plants of each seed type. Each pot was placed on top of an inverted, empty 5-inch pot to eliminate cross-contamination on the greenhouse bench. All pots were saturated with the Fowells and Krauss's solution. The pots were watered daily and saturated twice a week with Peter's Lo Phos Fertilizer.

Each of the six fungi being tested (C. geophilum, P. tinctorius, A. hygrometricus, R. roseolus, S. aurantium and A. muscaria) was used as inoculum for the onion seeds. Only Cenococcum geophilum and Pisolithus tinctorius were used to inoculate the corn, wheat, and asparagus seeds.

Inoculated plants were grown under normal greenhouse conditions with the day length extended to 12 hours with incandescent light bulbs.

After 8 weeks, plants were periodically removed from the greenhouse and the roots and perlite in which they were grown was examined as follows. Roots were individually washed in distilled water until free of perlite. The perlite that the plant grew in was agitated in 1 liter of distilled water and filtered along with the root washings through one layer of cheesecloth. This filtrate was then refiltered through nested 230 mesh and 325 mesh sieves. The retentate on the 325 mesh sieve was saved and examined microscopically. The cleaned roots were cleared and stained using the procedure of Kormanik et al. (Kormanik, Bryan and Schultz, Can. J. Microbiol. 26:536-538 (1980)). This procedure allows the microscopic visualization of fungi and fungal propagules within the intact root.

The roots of the plants that were examined after 8 weeks in the greenhouse showed no apparent internal fungal infection. However, mycelia were present on the exterior of the roots. The perlite washings revealed the presence of microsclerotia in every instance. When plants were similarly examined after 16 weeks, endomycorrhizal infection was well established in plants treated with all six fungal inoculants, and numerous microsclerotia were present on and around the roots. Root samples from plants grown 16 weeks or more were excised and fixed, dehydrated, and embedded in epoxy resin according to methods in Pizzolato, T. D., 1978, "A tannic acid-ferric chloride-toluidine blue stain for wood amyloplasts embedded in epoxy resin," Forest Science 24:49-51. The fixed samples were subsequently stained in Alsops reagent for 1 minute at 130°C by the methods of Alsop (1974) and Pizzolato (1984) (Alsop, D., 1974, "Rapid single solution polychrome and staining of semithin epoxy sections by polyethylene glycol 200 as a stain solvent," Stain Technology 49:265-272; Pizzolato, 1984, "Vascular system of the fertile floret of Anthoxanthum odoratum L.," Botanical Gazette 145(3):358-371). The light microscope sections of internal root cells obtained with these procedures revealed the presence of intercellular and intracellular hyphae, vesicles, and spore-like structures with all fungi and plants tested. Control plants yielded no evidence of infection or microsclerotia.

An examination of the fungal inocula used to infect the plants showed the presence of fungal hyphae that contained many swollen areas characteristic of the initials of sclerotia and microsclerotia. The inocula also contained spore-like structures. While microsclerotia have been observed in comparable inocula of the present invention, none were observed here. The failure to observe sclerotia or microsclerotia is not dispositive of their presence; it is not known how well these structures survive the preparation procedure for microscopic observation.

Results of these studies indicate that inocula produced from ectomycorrhizal fungi by procedures outlined above can be used to induce mycorrhizal infection in herbaceous plants. Furthermore, all the inocula produced were shown to form mycorrhizae with loblolly pine seedlings.

### EXAMPLES 7-19

### A. Preparation of Inoculum

The preparation of inocula as in Examples 1-6 was substantially repeated except that different fungal species were employed and a different agar medium was used for the initial growth incubation. Selected fungi (Athelia neuhoffii, Boletinellus merulioides, Hebeloma anthracophilum, Hebeloma crustuliniforme, Paxillus involutus, Piloderma bicolor, Rhizopogon nigrescens, Scleroderma albidum, Scleroderma polyrhizum, Suillus cothurnatus, Alpova pachyploeus, Boletus punctipes, and Lactarius deliciosus) were axenically grown from mycelia for four weeks at 24°C in the dark in petri plates on a modification of Melin and Rama Das nutrient agar (Melin and Rama Das "Influence of root metabolites on the growth of tree mycorrhizal fungi" Physiol. Plant. 7:851-858 (1954)).

**TABLE IV**

| Formulation of Modified Melin and Rama Das Agar | |
|---|---|
| KH₂PO₄ | 1.0 gm |
| MgS0₄.7H₂O | 0.5 gm |
| NH₄ Tartrate | 5.0 gm |
| ZnSO₄ (1:500) | 0.5 ml |
| Fe Citrate (1%) | 0.5 ml |
| Thiamin | 50.0 ug (microgram) |
| Glucose | 20.0 gm |
| Agar | 10.0 gm |
| Distilled H₂O | to 1000 ml |

Subsequently, 0.5 cm plugs were cut from the periphery of the fungal colonies and two plugs from each fungus were axenically added to a jar containing 100 ml of strained perlite and 55 ml of Fowells and Krauss's modified nutrient solution (Table III). The jars containing the perlite and nutrient solution had previously been autoclaved at 120°C and 15 lbs. pressure for 20 minutes. The inoculated jars were incubated for 3 months in the dark at 24°C.

### B. Ectomycorrhizal Infection in Asparagus Plants

Asparagus seeds that had been soaked in distilled water overnight were washed for one minute in 1% HgCl₂ with 1 ml of Tween 20 per liter and rinsed three times with sterile distilled water. Seeds were then axenically placed in sterile petri plates containing moistened filter paper and incubated in the dark at 24°C. Six days later, the germinated seeds were planted in 4-inch pots filled with strained horticultural grade perlite. A hole was dug 3 cm from the surface, and one teaspoon full of the fungus inoculum was put in the hole with one germinated seed placed on top of the inoculum and covered with perlite. Ten pots were prepared for each fungus being tested along with twenty control plants. Each pot was placed on top of an inverted empty 5 inch pot to eliminate contamination on the greenhouse bench. All pots were then saturated with the Fowells and Krauss's solution. The pots were watered daily and saturated twice a week with Peter's Lo Phos Fertilizer.

Inoculated plants were grown under normal greenhouse conditions. After 4 months, plants were removed from the greenhouse and the roots and perlite in which they were grown were examined as described above.

An examination of the fungal innocula used to infect the plants showed the presence of fungal hyphae that contained many swollen areas characteristic of the initials of sclerotia and microsclerotia. The inocula also contained spore-like structures.

Each of these fungal inocula forms mycorrhizae with Virginia pine or Loblolly pine in axenic culture.

Results of these studies indicate that inocula produced from ectomycorrhizal fungi by procedures outlined above can be used to induce mycorrhizal infection in herbaceous plants.

In view of the present specification, various additions, modifications, and omissions will be obvious to those skilled in the art and are within the invention as claimed below.

## Claims

1. The process of producing a mycorrhizal inoculant of a normally ectomycorrhizal fungi for use with either woody or herbaceous plants, comprising the steps of:
obtaining mycelia of an ectomycorrhizal fungus,
contacting a propagule of said mycelia with a growth medium and a particulate carrier, and
maintaining said contact in the absence of living plant matter in darkness and without aeration for a period of time effective to stress the mycelia, inducing it to produce at least microsclerotia or its initials among said mycelia, and
obtaining an ectomycorrhizal inoculum containing at least microsclerotia or the initials of microsclerotia, which inoculum is capable of producing intracellular hyphae when contacted with herbaceous plants.

2. The process of claim 1, wherein said obtained inoculum is effective to form mycorrhizal symbiotic associations with the roots of woody plants.

3. The process of claim 1, wherein said maintaining is conducted for about three months at about room temperature.

4. The process of claim 1, wherein said mycelia of selected ectomycorrhizal fungi are from the fungus species consisting of Rhizopogon roseolus, Pisolithus tinctorius, Amanita muscaria, Astraeus hygrometricus, Cenococcum geophilum, Scleroderma aurantium, Athelia neuhoffii, Boletinellus merulioides, Hebeloma anthracophilum, Hebeloma crustuliniforme, Paxillus involutus, Piloderma bicolor, Rhizopogon nigrescens, Scleroderma albidum, Scleroderma polyrhizum, Suillus cothurnatus, Alpova pachyploeus, Boletus punctipes, and Lactarius deliciosus.

5. The process of claim 1, wherein said carrier is selected from the group consisting of perlite and modified clays.

6. The process of claim 5, wherein in said contacting and maintaining steps the weight ratio of said particulate carrier to said growth medium is at least 2:1.

7. The process of claim 6, wherein said carrier is perlite.

8. The process of claim 1 wherein the propagule used in the contacting step are obtained by axenically growing said mycelia in the dark in petri dishes containing a nutrient-containing solid medium.

9. The process of claim 8 wherein the contacting step comprises cutting plugs from said petri dishes on which said mycelia have been grown and axenically contacting the plugs with the growth medium and particulate carrier.

10. The process of claim 9 wherein said carrier is perlite and said solid medium is agar.

11. The method of claim 1 wherein the growth medium is Fowells and Krause' pine nutrient solution modified by the addition of glucose and thiamine.

12. A mycorrhizal inoculant for either woody or herbaceous plants produced from mycelia of ectomycorrhizal fungi in the absence of living plant matter, said inoculant containing at least microsclerotia or the initials of microsclerotia and being capable of producing intracellular hyphae when contacted with herbaceous plants.

13. The inoculant according to claim 12, wherein said inoculant is effective to form endomycorrhizal symbiotic associations with herbaceous plants and mycorrhizal symbiotic associations with woody plants.

14. The mycorrhizal inoculant of claim 13, wherein said mycelia are of at least one isolated and cultivated ectomycorrhizal fungi.

15. The ectomycorrhizal inoculant of claim 12, wherein said mycelia of ectomycorrhizal fungi are of the fungus selected from the group consisting of Rhizopogan roseolus, Pisolithus tinctorius, Amanita muscaria, Astraeus hygrometricus, Cenococcum geophilum, Scleroderma aurantium, Athelia neuhoffii, Boletinellus merulioides, Hebeloma anthracophilum, Hebeloma crustuliniforme, Paxillus involutus, Piloderma bicolor, Rhizopogon nigrescens, Scleroderma albidum, Scleroderma polyrhizum, Suillus cothurnatus, Alpova pachyploeus, Boletus punctines, and Lactarius deliciosus.

16. A herbaceous seedling in contact with an ectomycorrhizal inoculant produced from mycelia of at least one isolated and cultivated ectomycorrhizal fungi in the absence of living plant matter wherein at least microsclerotia or the initials of microsclerotia that produce intracellular hyphae of a mycorrhizal fungus are present in or on the roots of said seedling.

17. The herbaceous seedling of claim 16, wherein said seedling is selected from the group consisting of corn, wheat, onion and asparagus seedlings.

18. A method of growing plants having mycorrhizae associated with their roots comprising the steps of:
obtaining mycelia of an ectomycorrhizal, microsclerotia-producing fungus, contacting propagule of said mycelia with both a growth medium containing a nutrient solution comprising sugar and thiamine and a particulate carrier,
maintaining said contact in the absence of living plant matter in darkness and without aeration for a period of time effective to stress the mycelia, inducing it to produce at least microsclerotia or its initials,
obtaining an ectomycorrhizal inoculum from said mycelia which is capable of producing intracellular hyphae when contacted with herbaceous plants,
germinating a seed to produce a seedling,
contacting said seedling with said inoculum and a nutrient solution, and
obtaining plants having a symbiotic association with said fungi.

19. The method of claim 18 wherein the nutrient solution is Fowells and Krauss' pine nutrient solution and the growth medium is pH balanced.

## Patentansprüche

1. Verfahren zur Herstellung eines Mycorrhiza Inokulums von normalen Ectomycorrhiza-Pilzen zur Verwendung bei entweder Holzpflanzen oder krautartigen Pflanzen, umfassend die Stufen:
Erhalten von Myzelien eines Ectomycorrhiza-Pilzes,
Inkontaktbringen eines Brutkörpers des Myzeliums mit Wachstumsmedium und einem teilchenförmigen Träger, und
Aufrechterhalten des Kontaktes in Abwesenheit lebenden Pflanzenmaterials in der Dunkelheit und ohne Belüftung, während eines Zeitraums, der wirksam ist, die Myzelien zu stressen, so daß sie dazu veranlaßt werden, wenigstens Mikrosklerotien oder deren Vorläufer bei den Myzelien zu bilden und
Erhalten eines Ectomycorrhiza-Inokulums, welches wenigstens Mikrosklerotien oder die Vorläufer von Mikrosklerotien enthält, wobei das Inokulum in der Lage ist, intrazelluläre Hyphen zu bilden, wenn es in Kontakt mit krautartigen Pflanzen gebracht wird.

2. Verfahren nach Anspruch 1, dadurch **gekennzeichnet,** daß das erhaltene Inokulum wirksam ist, symbiontische Mycorrhiza-Verbindungen mit den Wurzeln von Holzpflanzen zu bilden.

3. Verfahren nach Anspruch 1, dadurch **gekennzeichnet,** daß das Aufrechterhalten während etwa 3 Monaten bei etwa Raumtemperatur durchgeführt wird.

4. Verfahren nach Anspruch 1, dadurch **gekennzeichnet,** daß die Myzelien der ausgewählten Ectomycorrhiza-Pilze von den Pilzarten, bestehend aus Rhizopogon roseolus, Pisolithus tinctorius, Amanita muscaria, Astraeus hygrometricus, Cenococcum geophilum, Scleroderma aurantium, Athelia neuhoffii, Boletinellus merulioides, Hebeloma anthracophilum, Hebeloma crustuliniforme, Paxillus involutus, Piloderma bicolor, Rhizopogon nigrescens, Scleroderma albidum, Scleroderma polyrhizum, Suillus cothurnatus, Alpova pachyploeus, Boletus punctipes und Lactarius deliciosus, stammen.

5. Verfahren nach Anspruch 1, dadurch **gekennzeichnet,** daß der Träger ausgewählt ist aus der Gruppe, bestehend aus Perlit und modifizierten Tonen.

6. Verfahren nach Anspruch 5, dadurch **gekennzeichnet,** daß in den Stufen des Inkontaktbringens und des Aufrechterhaltens das Gewichtsverhältnis von teilchenförmigem Träger zu Wachstumsmedium wenigstens 2:1 ist.

7. Verfahren nach Anspruch 6, dadurch **gekennzeichnet,** daß der Träger Perlit ist.

8. Verfahren nach Anspruch 1, dadurch **gekennzeichnet,** daß der bei der Stufe des Inkontaktbringens verwendete Brutkörper durch steriles Wachsen des Myzels im Dunkeln in Petrischalen, welche ein Nährstoff enthaltendes festes Medium enthalten, erhalten wurde.

9. Verfahren nach Anspruch 8, dadurch **gekennzeichnet,** daß die Stufe des Inkontaktbringens das Herausschneiden von Stückchen aus den Petrischalen, auf welchen die Myzelien kultiviert wurden, und das sterile Inkontaktbringen der Stückchen mit dem Wachstumsmedium und dem teilchenförmigen Träger umfaßt.

10. Verfahren nach Anspruch 9, dadurch **gekennzeichnet,** daß der Träger Perlit und das feste Medium Agar ist.

11. Verfahren nach Anspruch 1, dadurch **gekennzeichnet,** daß das Wachstumsmedium Fowells- und Krauss-Kiefernährstofflösung, modifiziert durch die Zugabe von Glucose und Thiamin, ist.

12. Mycorrhiza-Inokulum für entweder Holzpflanzen oder krautartige Pflanzen, hergestellt aus Myzelien von Ectomycorrhiza-Pilzen in Abwesenheit lebenden Pflanzenmaterials, wobei das Inokulum wenigstens Mikrosklerotien oder Vorläufer von Mikrosklerotien enthält und in der Lage ist, intrazelluläre Hyphen zu bilden, wenn es in Kontakt mit krautartigen Pflanzen gebracht wird.

13. Inokulum nach Anspruch 12, dadurch **gekennzeichnet,** daß das Inokulum wirksam ist, symbiontische Endomycorrhiza-Verbindungen mit Krautpflanzen und symbiotische Mycorrhiza-Verbindungen mit Holzpflanzen zu bilden.

14. Mycorrhiza-Inokulum nach Anspruch 13, dadurch **gekennzeichnet,** daß die Myzelien von wenigstens einem isolierten und kultivierten Ectomycorrhiza-Pilz stammen.

15. Ectomycorrhiza-Inokulum nach Anspruch 12, dadurch **gekennzeichnet,** daß die Myzelien der Ectomycorrhiza-Pilze von einem Pilz, ausgewählt aus der Gruppe, bestehend aus Rhizopogan roseolus, Pisolithus tinctorius, Amanita muscaria, Astraeus hygrometricus, Cenococcum geophilum, Scleroderma aurantium, Athelia neuhoffii, Boletinellus merulioides, Hebeloma anthracophilum, Hebeloma crustuliniforme, Paxillus involutus, Piloderma bicolor, Rhizopogon nigrescens, Scleroderma albidum, Scleroderma polyrhizum, Suillus cothurnatus, Alpova pachyploeus, Boletus punctipes und Lactarius deliciosus, stammen.

16. Krautkeimling in Kontakt mit einem Ectomycorrhiza-Inokulum, hergestellt aus Myzelien von wenigstens einem isolierten und kultivierten Ectomycorrhiza-Pilz, in Abwesenheit lebenden Pflanzenmaterials, wobei wenigstens Mikrosklerotien oder Mikrosklerotien-Vorläufer, die intrazellulär Hyphen eines Mycorrhiza-Pilzes bilden, in oder an den Wurzeln des Keimlings vorhanden sind.

17. Krautkeimling nach Anspruch 16, dadurch **gekennzeichnet,** daß der Keimling ausgewählt ist aus der Gruppe, bestehend aus Mais-, Weizen-, Zwiebel- und Spargelkeimlingen.

18. Verfahren zum Kultivieren von Pflanzen mit mit ihren Wurzeln verbundenem Mycorrhiza, umfassend die Stufen:
Erhalten von Myzelien eines Ectomycorrhiza-, Mikrosklerotien-bildenden Pilzes, Inkontaktbringen des Brutkörpers der Myzelien mit sowohl einem Wachstumsmedium, welches eine Nährstofflösung enthält, die Zucker und Thiamin umfaßt, als auch mit einem teilchenförmigen Träger,
Aufrechterhalten des Kontaktes in Abwesenheit lebenden Pflanzenmaterials in Dunkelheit und ohne Belüftung während eines Zeitraums, der wirksam ist, die Myzelien zu stressen, so daß sie wenigstens Mikrosklerotien oder ihre Vorläufer bilden,
Erhalten eines Ectomycorrhiza-Inokulums von den Myzelien, das in der Lage ist, intrazelluläre Hyphen zu bilden, wenn es in Kontakt mit krautartigen Pflanzen gebracht wird,
Keimen eines Samens, um einen Keimling herzustellen,
Inkontaktbringen des Keimlings mit dem Inokulum und einer Nährstofflösung und
Erhalten von Pflanzen, die eine symbiontische Verbindung mit dem Pilz haben.

19. Verfahren nach Anspruch 18, dadurch **gekennzeichnet,** daß die Nährstofflösung Fowells- und Krauss-Kiefernährstofflösung ist und das Wachstumsmedium einen ausgeglichenen pH hat.

## Revendications

1. Procédé de préparation d'un inoculant mycorrhizien d'un champignon normalement ectomycorrhizien à utiliser avec des plantes, soit ligneuses, soit herbacées, comprenant les étapes consistant à:
obtenir des mycélia d'un champignon ectomycorrhizien,
mettre un propagule desdits mycélia en contact avec un milieu de croissance et un support particulaire, et
maintenir ledit contact en l'absence de matière végétale vivante, dans l'obscurité et sans aération pendant un laps de temps efficace pour exciter les mycélia en les induisant à produire au moins des microsclérotes ou leurs précurseurs parmi lesdits mycélia, et
obtenir un inoculum ectomycorrhizien contenant au moins des microsclérotes ou les précurseurs de microsclérotes, ledit inoculum étant à même de produire des hyphes intracellulaires lors de sa mise en contact avec des plantes herbacées.

2. Procédé selon la revendication 1, dans lequel ledit inoculum obtenu est efficace pour former des associations par symbiose mycorrhizienne avec les racines de plantes ligneuses.

3. Procédé selon la revendication 1, dans lequel on procède audit maintien pendant environ 3 mois à une température correspondant approximativement à la température ambiante.

4. Procédé selon la revendication 1, dans lequel lesdits mycélia de champignons ectomycorrhiziens sélectionnés proviennent des espèces fongiques constituées par Rhizopogon roseolus, Pisolithus tinctorius, Amanita muscaria, Astraeus hygrometricus, Cenococcum geophilum, Scleroderma aurantium, Athelia neuhoffii, Boletinellus merulioides, Hebeloma anthracophilum, Hebeloma crustuliniforme, Paxillus involutus, Piloderma bicolor, Rhizopogon nigrescens, Scleroderma albidum, Scleroderma polyrhizum, Suillus cothurnatus, Alpova pachyploeus, Boletus punctipes et Lactarius deliciosus.

5. Procédé selon la revendication 1, dans lequel ledit support est choisi parmi le groupe constitué par la perlite et des argiles modifiées.

6. Procédé selon la revendication 5, dans lequel, dans lesdites étapes de mise en contact et de maintien, le rapport pondéral dudit support particulaire audit milieu de croissance s'élève à au moins 2:1.

7. Procédé selon la revendication 6, dans lequel ledit support est la perlite.

8. Procédé selon la revendication 1, dans lequel le propagule utilisé dans l'étape de mise en contact est obtenu en faisant croître, dans des conditions axéniques, lesdits mycélia dans l'obscurité dans des boîtes de Pétri contenant un milieu solide qui contient un nutriment.

9. Procédé selon la revendication 8, dans lequel l'étape de mise en contact comprend le fait de pratiquer une découpe dans les pastilles desdites boîtes de Pétri sur lesquelles on a fait croître lesdits mycélia et le fait de mettre les pastilles, dans des conditions axéniques, en contact avec le milieu de croissance et le support particulaire.

10. Procédé selon la revendication 9, dans lequel ledit support est la perlite et ledit milieu solide est la gélose.

11. Procédé selon la revendication 1, dans lequel le milieu de croissance est une solution nutritive de pin de Fowells et de Krauss modifiée par l'addition de glucose et de thiamine.

12. Inoculant mycorrhizien destiné à des plantes, soit ligneuses, soit herbacées, obtenu à partir de mycélia de champignons ectomycorrhiziens en l'absence de matière végétale vivante, ledit inoculant contenant au moins des microsclérotes ou les précurseurs des microsclérotes, et étant à même de produire des hyphes intracellulaires lorsqu'on le met en contact avec des plantes herbacées.

13. Inoculant selon la revendication 12, dans lequel ledit inoculant est efficace pour former des associations par symbiose endomycorrhizienne avec des plantes herbacées et des associations par symbiose mycorrhizienne avec des plantes ligneuses.

14. Inoculant mycorrhizien selon la revendication 13, dans lequel lesdits mycélia proviennent d'au moins un champignon ectomycorrhizien isolé et mis en culture.

15. Inoculant ectomycorrhizien selon la revendication 12, dans lequel lesdits mycélia des champignons ectomycorrhiziens proviennent des champignons choisis parmi le groupe constitué par Rhizopogon roseolus, Pisolithus tinctorius, Amanita muscaria, Astraeus hygrometricus, Cenococcum geophilum, Scleroderma aurantium, Athelia neuhoffii, Boletinellus merulioides, Hebeloma anthracophilum, Hebeloma crustuliniforme, Paxillus involutus, Piloderma bicolor, Rhizopogon nigrescens, Scleroderma albidum, Scleroderma polyrhizum, Suillus cothurnatus, Alpova pachyploeus, Boletus punctipes et Lactarius deliciosus.

16. Plantule herbacé mis en contact avec un inoculant ectomycorrhizien obtenu à partir de mycélia d'au moins un champignon ectomycorrhizien isolé et mis en culture, en l'absence de matière végétale vivante, dans lequel au moins des microsclérotes ou les précurseurs des microsclérotes qui produisent des hyphes intracellulaires d'un champignon mycorrhizien sont présents dans ou sur les racines dudit plantule.

17. Plantule herbacé selon la revendication 16, dans lequel ledit plantule est choisi parmi le groupe constitué par des plantules de maïs, de froment, d'oignons et d'asperges.

18. Procédé pour faire croître des plantes dont les racines sont associées à des mycorrhizes, comprenant les étapes consistant à:
obtenir des mycélia d'un champignon ectomycorrhizien qui produit des microsclérotes,
mettre en contact le propagule desdits mycélia à la fois avec un milieu de croissance contenant une solution nutritive contenant du sucre et de la thiamine, et avec un support particulaire,
maintenir ledit contact en l'absence de matière végétale vivante, dans l'obscurité et sans aération pendant un laps de temps efficace pour exciter les mycélia en les induisant à produire au moins des microsclérotes ou leurs précurseurs,
obtenir un inoculum ectomycorrhizien à partir desdits mycélia, qui est capable de produire des hyphes intracellulaires lors de sa mise en contact avec des plantes herbacées,
faire germer une semence pour obtenir un plantule,
mettre ledit plantule en contact avec ledit inoculum et avec une solution nutritive, et
obtenir des plantes possédant une association symbiotique avec lesdits champignons.

19. Procédé selon la revendication 18, dans lequel la solution nutritive est une solution nutritive de pin de Fowells et de Krauss, et le milieu de croissance est équilibré quant au pH.
